# EUROPEAN PATENT APPLICATION

(11) **EP 4 243 447 A1**
(43) Date of publication of application: **13.09.2023**
(21) Application number: 22161494.4
(22) Date of filing: 11.03.2022
(51) Int. Cl.: H04R 25/00

(54) **HEARING DEVICE SYSTEM AND METHOD FOR PERFORMING A TEST OF A HEARING DEVICE USER'S HEARING CAPABILITIES**

(30) Priority: 10.03.2022 DK PA202270096
(71) Applicant: GN Hearing A/S, 2750 Ballerup (DK)
(72) Inventor: KÄSBACH, Johannes, 2750 Ballerup (DK); KRAAK, Joris, 2750 Ballerup (DK); COX, Marco, 2750 Ballerup (DK)
(74) Representative: Zacco Denmark A/S

(57) **Abstract**

Disclosed is a hearing device system, a hearing device and a method for performing a test of a hearing device user's hearing capabilities for providing a hearing threshold curve representing the user's hearing capabilities to be used for compensating for a hearing loss of the user. The hearing device system comprises an acoustic output transducer for outputting audio signals in the ear of the user. The hearing device system comprises a tone generator for outputting a plurality of tones in the acoustic output transducer. A first set of the plurality of tones will be audible to the user and a second set of the plurality of tones will be inaudible to the user. The hearing device system comprises a user interface configured for receiving an indication from the user when a tone is audible. The hearing device system comprises a processing unit configured for: adaptively selecting the plurality of tones, and adaptively selecting an order of the plurality of tones. The adaptive selection of a new tone of the plurality of tones is based on: whether a predefined number of the preceding tones were audible or inaudible to the user, and an estimated probability of whether the new tone is audible or inaudible to user.

## Description

### FIELD

The present invention relates to a hearing device system, a hearing device and a method for testing a user's hearing capabilities for providing a hearing threshold curve representing the user's hearing capabilities for use in a hearing device for the user. More specifically, the disclosure relates to a hearing device system comprising an acoustic output transducer for outputting audio signals in the ear of the user, and a tone generator for outputting a plurality of tones in the acoustic output transducer, where a first set of the plurality of tones will be audible to the user and a second set of the plurality of tones will be inaudible to the user. The hearing device system further comprises a user interface configured for receiving an indication from the user when a tone is audible.

### BACKGROUND

The most common and basic way to quantify hearing impairment is to estimate the pure tone hearing threshold (HT), which is called pure tone audiometry (PTA). The conventional way to estimate the pure tone HT of a person is to incrementally approximate the lowest tone intensity that can still be perceived at a set of standard frequencies ranging from 250 Hz to 8 kHz using a staircase "up 5 dB - down 10 dB" approach. This simple approach however suffers from a number of drawbacks.

Thus, there is a need for an improved hearing device system, hearing device and method for testing a user's hearing capabilities.

### SUMMARY

Disclosed is a hearing device system for performing a test of a hearing device user's hearing capabilities for providing a hearing threshold curve representing the user's hearing capabilities to be used for compensating for a hearing loss of the user. The hearing device system comprises an acoustic output transducer for outputting audio signals in the ear of the user. The hearing device system comprises a tone generator for outputting a plurality of tones in the acoustic output transducer. A first set of the plurality of tones will be audible to the user and a second set of the plurality of tones will be inaudible to the user. The hearing device system comprises a user interface configured for receiving an indication from the user when a tone is audible. The hearing device system comprises a processing unit configured for: adaptively selecting the plurality of tones, and adaptively selecting an order of the plurality of tones. The adaptive selection of a new tone of the plurality of tones is based on: whether a predefined number of the preceding tones were audible or inaudible to the user, and an estimated probability of whether the new tone is audible or inaudible to the user.

According to an aspect, disclosed is a hearing device for compensating for a hearing loss of a user. The hearing device is configured to be worn in an ear of the user. The hearing device is further configured for performing a test of the user's hearing capabilities for providing a hearing threshold curve representing the user's hearing capabilities to be used for the hearing compensation. The hearing device comprises an acoustic output transducer for outputting audio signals in the ear of the user. The hearing device comprises a tone generator for outputting a plurality of tones in the acoustic output transducer. A first set of the plurality of tones will be audible to the user and a second set of the plurality of tones will be inaudible to the user. The hearing device comprises a user interface or a communication unit for receiving an indication from the user when a tone is audible. The hearing device comprises a processing unit configured for: adaptively selecting the plurality of tones, and adaptively selecting an order of the plurality of tones. The adaptive selection of a new tone of the plurality of tones is based on: whether a predefined number of the preceding tones were audible or inaudible to the user, and an estimated probability of whether the new tone is audible or inaudible to the user.

According to an aspect, disclosed is a method for testing a user's hearing capabilities for providing a hearing threshold curve representing the user's hearing capabilities for use in a hearing device for the user. The method being performed in the hearing device system. The hearing device system comprises an acoustic output transducer for outputting audio signals in the ear of the user. The hearing device system comprises a tone generator for outputting a plurality of tones in the acoustic output transducer. A first set of the plurality of tones will be audible to the user and a second set of the plurality of tones will be inaudible to the user. The hearing device system comprises a user interface configured for receiving an indication from the user when a tone is audible. The hearing device system comprises a processing unit configured for: adaptively selecting the plurality of tones, and adaptively selecting an order of the plurality of tones. The method comprises performing an adaptive selection of a new tone of the plurality of tones based on: whether a predefined number of the preceding tones were audible or inaudible to the user, and an estimated probability of whether the new tone is audible or inaudible to the user.

The most common and basic way to quantify hearing impairment is to estimate the pure tone hearing threshold (HT), which is called pure tone audiometry (PTA). The conventional way to estimate the pure tone HT of a person is to incrementally approximate the lowest tone intensity that can still be perceived at a set of standard frequencies ranging from 250 Hz to 8 kHz using a staircase "up 5 dB - down 10 dB" approach. This simple approach however suffers from a number of drawbacks including that: A) It does not provide an uncertainty measure for the estimated thresholds, making it hard to define an objective stopping criterion. B) It is difficult to exploit potential prior knowledge about the threshold curve that is being estimated. C) It does not use all information available in the data by assuming that the hearing thresholds at different frequencies are uncorrelated. This leads to redundant measurements being required to achieve the desired accuracy. D) Restricting the stimuli to a fixed set of standard frequencies requires additional assumptions to estimate the threshold at other frequencies. The most common solution to this is to assume piecewise linearity of the HT curve on a semi-logarithmic frequency scale, for example in an audiogram. An audiogram depicts the difference between a particular HT curve and the HT curve of a normal-hearing person.

The present invention comprises a probabilistic approach to the pure tone hearing threshold estimation problem that addresses these drawbacks. Taking a probabilistic approach allows accounting for uncertainty in the patient's responses as well as incorporating prior knowledge in a fundamental way.

The present invention may comprise probabilistically modelling the HT as a continuous function of frequency, rather than using multiple independent response models (psychometric functions) for a discrete set of standard frequencies. This may be by endowing the threshold curve with a Gaussian process (GP) prior.

It is an advantage of the present invention that it provides uncertainty bands on the resulting threshold estimate, which enables fundamental and objective stopping criteria. Moreover, the present invention allows one to find the optimal next stimulus based on the data processed so far, and stimuli at any frequency may be used. These properties reduce the number of trials required to achieve the desired accuracy. Minimizing the amount or required trials is important to reduce the cognitive burden on the patients.

The present invention takes a probabilistic approach to the pure-tone hearing threshold estimation problem. One can view the threshold estimation problem as a probabilistic inference problem. To that end, we put a Gaussian process prior on the hearing threshold curve. Moreover, according to the present invention, the user's responses to stimuli may be modelled by a probabilistic response model. This model describes how the user response depends on their hearing threshold and the presented test tone. Given the response model, approximate Bayesian inference techniques may be applied to approximate the posterior distribution of the hearing threshold. This may result in a "Bayesian pure-tone audiometry" method. The Bayesian pure-tone audiometry method may incrementally decrease the uncertainty about the users hearing threshold curve by repeating the following steps:
1. Use the current (uncertain) estimate of the HT curve to determine the 'optimal' (most informative) next test tone. This tone may or may not be restricted to a set of standard frequencies.
2. Play the test tone and collect the user's binary response: audible or non-audible.
3. Update the probabilistic estimate of the HT curve (i.e. approximate the posterior GP) based on the data collected so far.

These steps may be repeated until the uncertainty about the hearing threshold is sufficiently small. The fully probabilistic treatment of the estimation problem has some favourable properties:
An objective, information-theoretic criterion can be used to pick test tones such that the uncertainty is decreased as fast as possible. This minimizes the required number of trials to reach the desired accuracy.
Prior knowledge about the user's hearing threshold - for example based on age or medical history - can be leveraged to further decrease the number of required trials.
The complete threshold curve is inferred directly, instead of only inferring the thresholds at a set of standard frequencies.
The stopping criterium can be defined in an objective manner.
The method can be combined with other threshold estimation methods that take a probabilistic HT estimate as input.

According to an aspect, the present hearing device system is configured for performing a test of a hearing device user's hearing capabilities for providing a hearing threshold curve representing the user's hearing capabilities to be used for compensating for a hearing loss of the user.

According to an aspect, the present hearing device is configured for performing a test of the hearing device user's hearing capabilities for providing a hearing threshold curve representing the user's hearing capabilities to be used for compensating for a hearing loss of the user.

The hearing device system may comprise a hearing device configured to be worn in/at the ear of the user. The test may be performed when the user is wearing the hearing device. The test may be performed by the hearing device. After the test, the user may wear the hearing device on a daily basis for compensating for the user's hearing loss.

The test may be performed before starting to use the hearing device for the first time, and/or the test may be performed after the hearing device has been used for some time, e.g. as an update of the user's hearing threshold curve for updating the compensation for the hearing loss. The update may be performed at regular intervals.

The test may be performed by the user herself/himself without interaction from a hearing case professional. Alternatively, the test may be performed by the user with interaction from a hearing case professional. Alternatively, the test may be performed by a hearing case professional.

The term "hearing threshold curve" may be used interchangeably with the term "audiogram" in the present disclosure. However, an "audiogram" may imply additional restrictions on the hardware used according to certain standards.

According to an aspect, the hearing device system comprises an acoustic output transducer for outputting audio signals in the ear of the user. According to an aspect, the hearing device comprises an acoustic output transducer for outputting audio signals in the ear of the user. The acoustic output transducer may be a speaker, a loudspeaker, a receiver, and outrput transducer etc.

According to an aspect, the hearing device system comprises a tone generator for outputting a plurality of tones in the acoustic output transducer. According to an aspect, the hearing device comprises a tone generator for outputting a plurality of tones in the acoustic output transducer. A first set of the plurality of tones will be audible to the user and a second set of the plurality of tones will be inaudible to the user. Each tone of the plurality of tones may have a frequency, measured in e.g. Hz, and an intensity, measured in e.g. dB. The tone generator may be implemented in the processing unit, mentioned below, of the hearing device system or of the hearing device. The tone generator may be a separate tone generator of the hearing device system or of the hearing device.

According to an aspect, the hearing device system comprises a user interface configured for receiving an indication from the user when a tone is audible. The hearing device system may comprise a hearing device. The user interface may be arranged on the hearing device, which is configured to worn by the user during the test. The user interface may be a physical push-button on the hearing device. The hearing device system may comprise an electronic device, such as a smartphone or a remote control. The user interface may be arranged on the electronic device. The user interface may comprise a graphical user interface comprising an electronic or virtual affordance on which the user can indicate when a tone is audible.

According to an aspect, the hearing device comprises a user interface or a communication unit for receiving an indication from the user when a tone is audible. The user interface may be arranged on the hearing device, which is configured to worn by the user during the test. The user interface may be a physical push-button on the hearing device. The hearing device may be associated and/or connected to an electronic device, such as a smartphone or a remote control. The user interface may be arranged on the electronic device. The user interface may comprise a graphical user interface comprising an electronic or virtual affordance on which the user can indicate when a tone is audible.

As an alternative or addition to a user interface, the hearing device may comprise a communication unit for receiving an indication from the user when a tone is audible. The communication unit may be or may comprise a wireless transceiver. If the hearing device does not have a user interface, e.g. a push-button, then the indication from the user may be provided on a user interface of an associated and connected electronic device, and then received in the communication unit of the hearing device after being transmitted from the electronic device.

According to an aspect, the hearing device system comprises a processing unit configured for: adaptively selecting the plurality of tones, and adaptively selecting an order of the plurality of tones. The processing unit may be arranged in a hearing device of the hearing device system, where the user is wearing the hearing device during the test. The processing unit may be arranged in an electronic device of the hearing device system. The processing unit may be a functionality of a software application, a socalled app, of the electronic device. The proceesing unit may be arranged in or may be a functionality of a server of the hearing device system.

According to an aspect, the hearing device comprises a processing unit configured for: adaptively selecting the plurality of tones, and adaptively selecting an order of the plurality of tones.

The adaptive selection of a new tone of the plurality of tones is based on: whether a predefined number of the preceding tones were audible or inaudible to the user, and an estimated probability of whether the new tone is audible or inaudible to the user. Audible tones are indicated by the user on the user interface. Inaudible tones are detected as being not indicated by the user.

The estimated probability of whether the new tone is audible or inaudible to the user may be a fixed probability, a determined probability, a calculated probability, a predefined probability, an adjusted probability etc. The processing unit is configured to perform the adaptive selection of a new tone based on, among other, the estimated probability of whether the user can hear the new tone or not. The processing unit may perform the estimation of the probability, the probability may be estimated once or continuously.

A prior art audiometric process that tries to be optimal in an 'information sense', such as those powered by Bayesian Pure-Tone Audiometry (BPTA) which learns from both positive (audible tone) and negative (inaudible tone) responses from a user, may not be optimal from a 'user experience' perspective. This is due to such a process presenting tones 'at the estimated hearing threshold' of an individual. Repeatedly presenting tones 'at the estimated hearing threshold' leads to a significant probability of presenting multiple audible or inaudible tones in a row. This can be detrimental to the user experience, especially in the case of inaudible tones since the user might interpret it as the system not working (correctly) anymore.

This was observed during BPTA pilot tests performed by the applicant where one complaint was extended periods of silence between audible tones.

See for example the published background reference: Cox, M. and de Vries, B. 2016: 'A Bayesian binary classification approach to pure tone audiometry'.

According to the present invention, to improve the 'user experience' of audiometry processes powered by BPTA, the present invention may provide a couple of mechanisms that can be used to trade-off average convergence speed in terms of number of tones presented, for a similar accuracy, against the likelihood that a presented tone will result in a positive or a negative response. This makes it more or less likely that a tone will be audible relative to the mode that aims at 'maximum efficiency in terms of the number of iterations' while decreasing the efficiency of the process.

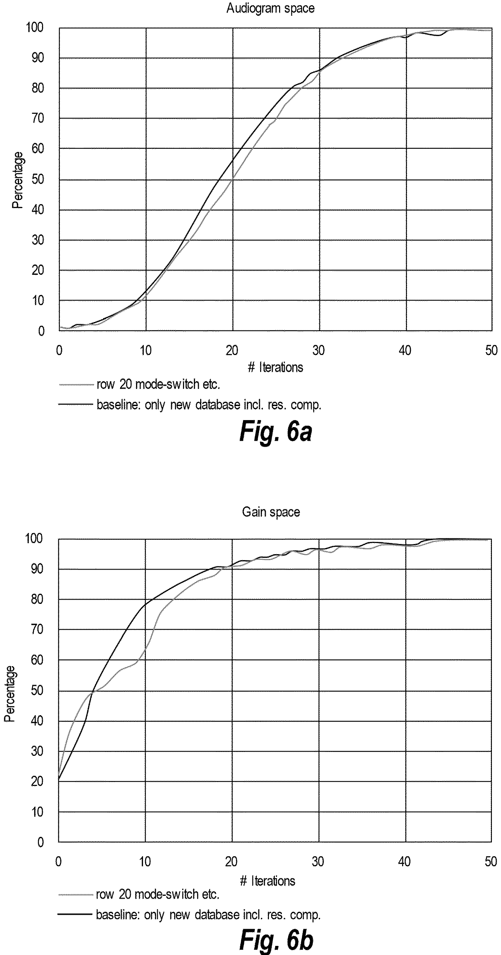

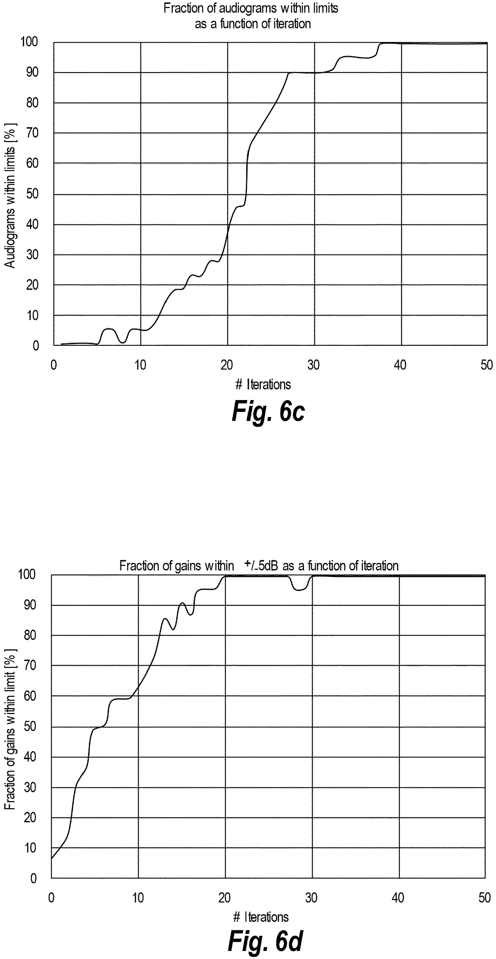

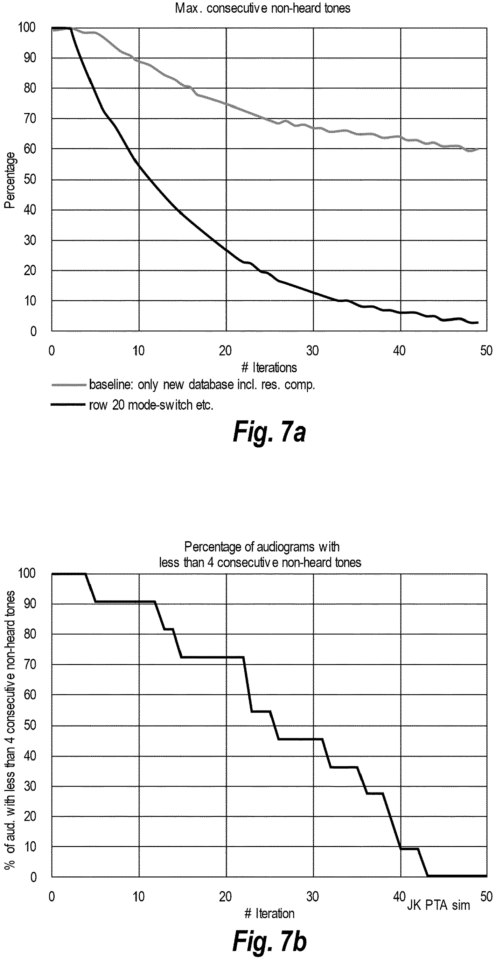

These mechanisms comprise: The motivation for the proposed method arises from the fact that long pauses in the procedure are associated with a bad user experience. Long pauses are caused when the method successively tests at or below the hearing threshold of that person: A non-heard tone will cause waiting time, hence the user might think the method is erroneous or not working correctly. With the present method it is an aim to have a good balance between heard and non-heard tones such that the user has a satisfying experience, e.g. not too long pauses, and at the same time not compromising accuracy in the method itself. The improvement can be explained by a plot. Consider Figures 6 and 7. Figure 6 shows the accuracy of the BPTA method as a function of iterations in audiogram space (left column) and gain space (right column), without taking the improved user experience into account (dark curve) and taken the user experience into account (bright curve). The accuracy is unaltered in both ways. However, the user experience is significantly improved which becomes apparent when considering Figure 7. Figure 7 shows the consecutive number of non-heard tones, percentage of less than 4 consecutive non-heard tones, as a function of number of iterations, simply a measure of waiting time. A higher value is desirable here, ensuring that there are not too long pauses due to non-heard tones. Here the bright curve, being the user experience improvement, overrules the dark curve, being neglecting the user experience, suggesting that the present method provides the desired user experience.

The mechanisms further comprise: Adjusting the set of frequencies at which BPTA is allowed to suggest tones. In some case, this is restricted to the 'standard audiometric frequencies'.

The algorithm may only use tones at 10 audiometric frequencies (250, 500, 750, 1000, 1500, 2000, 3000, 4000, 6000 and 8000 Hz). However, in some cases, e.g., using BPTA for a specific hearing device type, the algorithm may omit frequencies at 250 Hz and at 8 kHz due to unreliable measurements at these frequencies, e.g. acoustic leakage at low frequencies and too high individual ear shapes causing too high variations at high frequencies.

The BPTA method is intelligent in the way that it may always choose the frequency and sound intensity that will provide the most information regarding converging to the true audiogram of the person under test based on all previous trials.

The 'standard audiometric frequencies' may be a set of standardized frequencies that are common in audiometric use, hence: 125, 250, 500, 750, 1000, 1250, 1500, 2000, 3000, 4000, 6000 and 8000 Hz. A subset of these frequencies may be selected prior to performing the BPTA method.

The first task of the BPTA method may be to adjust the probability that a tone will be audible given the estimate of the hearing threshold, which affects the intensity of the suggested tones. By default, this may be set to '0', which indicates to use intensities that match the estimated hearing threshold, the estimate being derived, e.g., from an audiogram taken prior to performing the BPTA method.

There may be two parts of the BPTA process, and these are described in the following. The first part may address the 'housekeeping tasks", e.g. ensuring that the user types in information related to age and gender prior to the audiometric process. This may provide the starting point of the audiometric process. A corresponding audiogram may be chosen based on age and gender if a personal audiogram is not available. This information may be extracted from a Gaussian modelling process on a large database, such as a database comprising about 80000 audiograms. The second part may address probabilities of the hearing thresholds for each of the subset of standardized frequencies. By default, the BPTA method may always propose a signal level near the expected hearing threshold of that person under test for a certain frequency. However, with the present improved user experience (UX) method, different probabilities can be selected. If the probability is set low, the algorithm may skew the suggested signal level towards the inaudible part of the hearing threshold. If the probability is set high, it may skew the suggested signal level towards the audible part of the hearing threshold. Note that the BPTA method may gain more new information the closer to the hearing threshold tones are tested. Therefore, there may always be the trade-off to consider of gaining new information with new tones but at the same time maintaining a good user experience, hence a good balance between heard and non-heard tones. Such trade-off was empirically evaluated by the applicant and led to the examples of numbers below.

The recommended BPTA parameter settings for a specific hearing device may be:
- x = 10,
- y1 = z1 = 5 and a corresponding audibility probability of 15%,
- y2 = z2 = 2 and a corresponding audibility probability of 85%.

The different ways of manipulating these mechanisms may be referred to as 'mode-switching'. Three strategies for this have been implemented in applications used for pilot testing by the applicant and can be applied at the same time:
The modes may be defined in the five variables x, y1, z1, y2 and z2. In general, the modes may refer to:
   - Default mode, i.e., BPTA may be working as described in the original reference of the method described in Cox, M. and de Vries, B. 2016.
   - Improved UX mode, i.e., one or more of the three strategies may be active with the goal to improve user experience without compromising on accuracy.

A first mode-switching strategy may cover variable x:
For the first x tones the focus may be on those frequencies important for speech, e.g 1000-4000Hz. This restriction may be released at the x+1st tone.
Hence, the algorithms may only select the frequencies 1000, 1500, 2000, 3000 or 4000 Hz. As stated, the purpose may be to start with frequencies that are the most important for speech perception.

A second mode-switching strategy may cover the variables y1 and z1 and may address the situation where there are too many consecutive audible tones:
In order to "try to force an inaudible tone", the audibility probability of the proposed tones may be adjusted to 15% after y1 consecutive, audible tones, and the subset of frequencies which the method is allowed to propose may be reduced to only those frequencies for which an inaudible response has not yet been observed after z1 consecutive audible tones.

A third mode-switching strategy may cover the variables y2 and z2 and may address the situation when there are too many consecutive inaudible tones:
In order to "try to force an audible tone", the audibility probability of the proposed tones may be adjusted to 85% after y2 consecutive, inaudible tones, and the subset of frequencies which the method is allowed to propose may be reduced to those frequencies for which an audible response has not yet been observed after z2 consecutive inaudible tones.

For the first x tones the focus may be on those frequencies important for speech, e.g. 1000-4000Hz. This restriction may be released at the x+1st tone.

The term 'focus' may imply that "the first x tones has to have a frequency in the range 1000-4000 Hz". Thus, the BPTA system may be required to propose tones with frequencies in the range 1000-4000 Hz in the first x steps. After x tones, this requirement may be lifted so tones with lower or higher frequencies are also allowed. This requirement ensures that the hearing threshold level may be first estimated at the frequencies that are important for speech.

After y1 number of audible tones, all frequencies may be removed from the set allowed for frequency selection for which no inaudible responses have been recorded. The frequency set may, however, be restored to all frequencies configured for the process after an inaudible response has been obtained.

The value of the parameter y1 may be independent of the method description. For a specific hearing device, the recommended value of y1=5 may be based on empirical optimization through a set of simulations. The applicant found that setting y1=5 yielded the best performance in the simulations. During the simulations, this value provided a good trade-off between triggering the method when it was beneficial while not triggering it too often.

Frequencies may be removed from the set if no inaudible responses have been recorded. The main reason being that since those frequencies can be heard by the user it is unnecessary to repeat them, thus saving time. In this context, "the set" may relate to the set of frequencies that BPTA to choose from to propose the next test tone. The method may keep track of a set of frequencies that BPTA can choose from when proposing the next tone to test. If a frequency is removed from the set, BPTA may not propose tones with that frequency.

By default, the frequency set, i.e. the set of frequencies that BPTA can choose from when proposing tones, may contain the standard audiometric frequencies. However, the various mechanisms in the described method may adaptively add or remove frequencies from this set. If the frequency set is restored, it may be set back to the default (= standard audiometric frequencies).

After y2 number of inaudible tones all frequencies may be removed from the set allowed for frequency selection for which no audible responses have been recorded. The main reason being that since those frequencies cannot be heard by the user it is unnecessary to repeat them, thus saving time. The frequency set may be restored to all frequencies configured for the process after an audible response has been obtained.

The parameter y2 may be chosen in a manner similar to y1, through empirical optimization by simulations. In general, the parameters can be chosen by hand, optimized through simulations, or optimized through real-world experiments.

After z1 number of audible tones, the 'audibility probability' may be reduced closer to 0%, but not at 0%, to try and force an inaudible response. Once an inaudible response has been obtained, the audibility probability may be reset to the default selected for the process.

Both values of parameters z1 and z2 may be obtained through empirical optimization. They do not need to be equal, but testing has revealed that setting them to the same value provides the best performance. This is expected since both parameters determine when a mechanism is triggered to "force" an audible or inaudible tone.

After z2 number of inaudible tones, the 'audibility probability' may be increased closer to 100% to try and force an audible response. Once an audible response has been obtained, the audibility probability may be reset to the default probability selected for the process. This is also empirically determined. The audibility probabilities related to "inaudible" and "audible" tones are informed choices to increase the likelihood of the tone being evaluated at the desired audibility while still providing information to the system. Setting these to, say, 1% and 100% respectively, would make it very unlikely for the system to learn. The default setting of "the estimated threshold", may be the "most informative". The further evaluated tones deviate from this value, the less "informative" they may be.

The method is a tradeoff between accuracy and user experience given a fixed budget of stimuli, i.e., how many stimuli is a user willing to evaluate during a session. Typically, the same accuracy can also be acquired by extending the budget. However, that may arguably decrease the user experience as the procedure requires more investment from the user regarding time, attention, and patience. These methods are specifically designed for automated procedures in situations where a limited time investment is targeted to be required from an end-user, but where the completion of the procedure is a necessity for accurate functioning of systems further down the pipeline, e.g. the determination of compressor settings in a hearing instrument. In user trials, the applicant has observed people to get bored/disinterested when they don't hear enough variation in the tones, e.g. they always hear a tone, or they start doubting whether the system even works because they never hear a tone.

The improved method is intended as an extension for automated, autonomously operating systems interacting with end-users through a 'propose/observe' loop, learning about aspects of their behavior or properties, e.g., determination of a hearing threshold. It is specifically intended for systems designed to select proposals in a mathematically optimal way, instead of systems intended to account for user error by expending more effort in interacting with a user, such as the more traditional Hughson-Westlake procedure for obtaining a hearing threshold.

Although the method as described here may be specific to BPTA, it is essentially very generic in the way that it concerns the adjustment of the stimulus selection mechanism of an automated system based on the observations by an end-user. The multiple methods fit within a more generic framework with other strategies being conceivable but not deemed necessary within the context of simple tone selection.

In some embodiments, the adaptive selection of a new tone is based on between one and five preceding tones, preferably two preceding tones, being inaudible to the user before a probable audible tone is selected.
In some embodiments, the adaptive selection of a new tone is based on two preceding tones being inaudible to the user before a probable audible tone is selected. It is an advantage that only two consequtive tones are inaudible to the user because otherwise the user may think the test is not working if there are too many inaudibile tones. A probable audible tone may be a tone expected to be audible or a tone likely to be audible.

In some embodiments, the adaptive selection of a new tone is based on between two and eight preceding tones, preferably five preceding tones, being audible to the user before a probable inaudible tone is selected.
In some embodiments, the adaptive selection of a new tone is based on five preceding tones being audible to the user before a probable inaudible tone is selected. It is an advantage to have maximum 5 consequtive audible tones because otherwise the user may think that the test is boring and that he/she has no hearing loss. A probable inaudible tone may be a tone expected to be inaudible or a tone likely to be inaudible.

In some embodiments, the estimated probability of whether the new tone is audible to the user is between about 75% and about 95%, preferably about 85%, probability.
In some embodiments, the estimated probability of whether the new tone is audible to the user is about 85% probability. Thus, the estimated probability of whether the new tone is audible to the user may be about 70%-100% probability, or about 75%-95% probability, or about 80%-90% probability, such as about 85% probability.

In some embodiments, the estimated probability of whether the new tone is inaudible to the user is between about 5% and about 25%, preferably about 15% probability.
In some embodiments, the estimated probability of whether the new tone is inaudible to the user is about 15% probability. Thus, the estimated probability of whether the new tone is inaudible to the user may be about 0%-30% probability, or about 5%-25% probability, or about 10%-20% probability, such as about 15% probability

In some embodiments, the plurality of tones are selected from a set of audiometric frequencies in a range from about 200 Hz to about 10 kHz, preferably including the frequencies 250 Hz, 500 Hz, 750 Hz, 1000 Hz, 1500 Hz, 2000 Hz, 3000 Hz, 4000 Hz, 6000 Hz, and 8000 Hz.
In some embodiments, the plurality of tones are selected from a set of audiometric frequencies including 250 Hz, 500 Hz, 750 Hz, 1000 Hz, 1500 Hz, 2000 Hz, 3000 Hz, 4000 Hz, 6000 Hz, and 8000 Hz.

In some embodiments, the plurality of tones in a mid-frequency trial of the test are 10 tones selected from a subset of audiometric frequencies important for speech in the range from about 1kHz to about 4 kHz, preferably including the frequencies 1000 Hz, 1500 Hz, 2000 Hz, 3000 Hz, 4000 Hz.
In some embodiments, the plurality of tones in a mid-frequency trial of the test are 10 tones selected from a subset of audiometric frequencies important for speech, including 1000 Hz, 1500 Hz, 2000 Hz, 3000 Hz, 4000 Hz. It is an advantage to use only about 10 tones in the mid-frequency trial of the test, because this will provide a good test result while keeping the test time down. The test may be performed within a few minutes.

In some embodiments, the plurality of tones in the complete test is dependent of a predetermined confidence interval of the test, preferably is at least 20 tones.
In some embodiments, the plurality of tones in the complete test is at least 20 tones. In some embodiments, the plurality of tones in the complete test is 20-30 tones. In some embodiments, the plurality of tones in the complete test is less than 50 tones. Thus, the minimum of 20 tones may be a hard confidence limit. The test may converge at about 30-50 tones.

In some embodiments, the hearing threshold curve representing the user's hearing capabilities is estimated by Bayesian pure tone audiometry (BPTA).

In some embodiments, the Bayesian pure tone audiometry (BPTA) comprises a probabilistic approach to estimate the hearing threshold of the user based on a probabilistic model of the hearing threshold curve, consisting of a weighted combination of Gaussian process components that represent typical hearing threshold curves, the components being trained on a database of hearing threshold curves, where information including the user's age and gender is used to compute the underlying weights of the components, and where the BPTA estimates the user's hearing threshold curves as a smooth continuous function of frequency versus hearing threshold.

In some embodiments, the hearing device system is configured for performing the test of the user's hearing capabilities for providing the hearing threshold curve representing the user's hearing capabilities by initiation from the user and without assistance from a hearing care professional. Thus the test can be performed by the user him/herself without any help from a professional, socalled self-fitting. The user may simply download an app or select a certain function in an existing app for the hearing device, for starting the test. The app may be on an associated electronic device. The self-fitting procedure may alternatively and/or additionally be a special feature accessible from a dedicated hearing device remote control.

In some embodiments, the processing unit is configured for generating the hearing threshold curve representing the user's hearing capabilities to be used for the hearing compensation when the plurality of tones has been outputted according to the adaptive selection.

In some embodiments, the hearing device system comprises a hearing device configured to be worn in an ear of the user for compensating for the hearing loss of the user, and wherein the hearing device comprises the acoustic output transducer for outputting audio signals in the ear of the user. The hearing device may be used for both the test and for the daily wear for proving hearing compensation to the user. The hearing device may therefore comprise the acoustic output transducer for outputting audio signals in the ear of the user during the test and for outputting processed audio signals for providing hearing compensation during daily wear of the hearing device. Optionally, the hearing device may also comprise the tone generator, the user interface, and/or the processing unit.

In some embodiments, the processing unit is a first processing unit of the hearing device or a second processing unit of an electronic device. Thus, the functionality of the processing unit is a part of the hearing device system, and it may be built into the hearing device, or may be performed by an app.

In some embodiments, the user interface is provided at the hearing device, at an auxiliary device, and/or at an electronic device comprising a communication unit for communicating with a hearing device system communication unit. The electronic device may be e.g. a smartphone, a tablet, a pc etc. The electronic device may have a user interface where the user can indicate when a tone is audible, e.g. a touch screen or a button. The electronic device or the auxiliary device may be a hardware remote control or a charging unit for the hearing device. The electronic device or the auxiliary device may be a dedicated hardware remote control, e.g., built into the hearing device charger. The communication unit of the hearing device/electronic device/auxiliary device and hearing device system communication unit may e.g. comprise a wireless communication unit and an antenna. The communication may thus be wirelss communication e.g. using Bluetooth (BT) or other wireless communication. Alternatively, the communication may be wired.

In an embodiment, a hearing device is configured to be worn by a user. The hearing device may be arranged at the user's ear, on the user's ear, over the user's ear, in the user's ear, in the user's ear canal, behind the user's ear and/or in the user's concha, i.e., the hearing device is configured to be worn in, on, over and/or at the user's ear. The user may wear two hearing devices, one hearing device at each ear. The two hearing devices may be connected, such as wirelessly connected and/or connected by wires, such as a binaural hearing aid system.

The hearing device may be a hearable such as a headset, headphone, earphone, earbud, hearing aid, a personal sound amplification product (PSAP), an over-the-counter (OTC) hearing device, a hearing protection device, a one-size-fits-all hearing device, a custom hearing device or another head-wearable hearing device. Hearing devices can include both prescription devices and non-prescription devices.

The hearing device may be embodied in various housing styles or form factors. Some of these form factors are Behind-the-Ear (BTE) hearing device, Receiver-in-Canal (RIC) hearing device, Receiver-in-Ear (RIE) hearing device or Microphone-and-Receiver-in-Ear (MaRIE) hearing device. These devices may comprise a BTE unit configured to be worn behind the ear of the user and an in the ear (ITE) unit configured to be inserted partly or fully into the user's ear canal. Generally, the BTE unit may comprise at least one input transducer, a power source and a processing unit. The term BTE hearing device refers to a hearing device where the receiver, i.e. the output transducer, is comprised in the BTE unit and sound is guided to the ITE unit via a sound tube connecting the BTE and ITE units, whereas the terms RIE, RIC and MaRIE hearing devices refer to hearing devices where the receiver may be comprise in the ITE unit, which is coupled to the BTE unit via a connector cable or wire configured for transferring electric signals between the BTE and ITE units.

Some of these form factors are In-the-Ear (ITE) hearing device, Completely-in-Canal (CIC) hearing device or Invisible-in-Canal (IIC) hearing device. These hearing devices may comprise an ITE unit, wherein the ITE unit may comprise at least one input transducer, a power source, a processing unit and an output transducer. These form factors may be custom devices, meaning that the ITE unit may comprise a housing having a shell made from a hard material, such as a hard polymer or metal, or a soft material such as a rubber-like polymer, molded to have an outer shape conforming to the shape of the specific user's ear canal.

Some of these form factors are earbuds, on the ear headphones or over the ear headphones. The person skilled in the art is well aware of different kinds of hearing devices and of different options for arranging the hearing device in, on, over and/or at the ear of the hearing device wearer. The hearing device (or pair of hearing devices) may be custom fitted, standard fitted, open fitted and/or occlusive fitted.

In an embodiment, the hearing device may comprise one or more input transducers. The one or more input transducers may comprise one or more microphones. The one or more input transducers may comprise one or more vibration sensors configured for detecting bone vibration. The one or more input transducer(s) may be configured for converting an acoustic signal into a first electric input signal. The first electric input signal may be an analogue signal. The first electric input signal may be a digital signal. The one or more input transducer(s) may be coupled to one or more analogue-to-digital converter(s) configured for converting the analogue first input signal into a digital first input signal.

In an embodiment, the hearing device may comprise one or more antenna(s) configured for wireless communication. The one or more antenna(s) may comprise an electric antenna. The electric antenna may be configured for wireless communication at a first frequency. The first frequency may be above 800 MHz, preferably a wavelength between 900 MHz and 6 GHz. The first frequency may be 902 MHz to 928 MHz. The first frequency may be 2.4 to 2.5 GHz. The first frequency may be 5.725 GHz to 5.875 GHz. The one or more antenna(s) may comprise a magnetic antenna. The magnetic antenna may comprise a magnetic core. The magnetic antenna may comprise a coil. The coil may be coiled around the magnetic core. The magnetic antenna may be configured for wireless communication at a second frequency. The second frequency may be below 100 MHz. The second frequency may be between 9 MHz and 15 MHz.

In an embodiment, the hearing device may comprise one or more wireless communication unit(s). The one or more wireless communication unit(s) may comprise one or more wireless receiver(s), one or more wireless transmitter(s), one or more transmitter-receiver pair(s) and/or one or more transceiver(s). At least one of the one or more wireless communication unit(s) may be coupled to the one or more antenna(s). The wireless communication unit may be configured for converting a wireless signal received by at least one of the one or more antenna(s) into a second electric input signal. The hearing device may be configured for wired/wireless audio communication, e.g. enabling the user to listen to media, such as music or radio and/or enabling the user to perform phone calls.

In an embodiment, the wireless signal may originate from one or more external source(s) and/or external devices, such as spouse microphone device(s), wireless audio transmitter(s), smart computer(s) and/or distributed microphone array(s) associated with a wireless transmitter. The wireless input signal(s) may origin from another hearing device, e.g., as part of a binaural hearing system and/or from one or more accessory device(s), such as a smartphone and/or a smart watch.

In an embodiment, the hearing device may include a processing unit. The processing unit may be configured for processing the first and/or second electric input signal(s). The processing may comprise compensating for a hearing loss of the user, i.e., apply frequency dependent gain to input signals in accordance with the user's frequency dependent hearing impairment. The processing may comprise performing feedback cancelation, beamforming, tinnitus reduction/masking, noise reduction, noise cancellation, speech recognition, bass adjustment, treble adjustment and/or processing of user input. The processing unit may be a processor, an integrated circuit, an application, functional module, etc. The processing unit may be implemented in a signal-processing chip or a printed circuit board (PCB). The processing unit may be configured to provide a first electric output signal based on the processing of the first and/or second electric input signal(s). The processing unit may be configured to provide a second electric output signal. The second electric output signal may be based on the processing of the first and/or second electric input signal(s).

In an embodiment, the hearing device may comprise an output transducer. The output transducer may be coupled to the processing unit. The output transducer may be a receiver. It is noted that in this context, a receiver may be a loudspeaker, whereas a wireless receiver may be a device configured for processing a wireless signal. The receiver may be configured for converting the first electric output signal into an acoustic output signal. The output transducer may be coupled to the processing unit via the magnetic antenna. The output transducer may be comprised in an ITE unit or in an earpiece, e.g. Receiver-in-Ear (RIE) unit or Microphone-and-Receiver-in-Ear (MaRIE) unit, of the hearing device. One or more of the input transducer(s) may be comprised in an ITE unit or in an earpiece.

In an embodiment, the hearing device may comprise a digital-to-analogue converter configured to convert the first electric output signal, the second electric output signal and/or the wireless output signal into an analogue signal.

In an embodiment, the hearing device may comprise a vent. A vent is a physical passageway such as a canal or tube primarily placed to offer pressure equalization across a housing placed in the ear such as an ITE hearing device, an ITE unit of a BTE hearing device, a CIC hearing device, a RIE hearing device, a RIC hearing device, a MaRIE hearing device or a dome tip/earmold. The vent may be a pressure vent with a small cross section area, which is preferably acoustically sealed. The vent may be an acoustic vent configured for occlusion cancellation. The vent may be an active vent enabling opening or closing of the vent during use of the hearing device. The active vent may comprise a valve.

In an embodiment, the hearing device may comprise a power source. The power source may comprise a battery providing a first voltage. The battery may be a rechargeable battery. The battery may be a replaceable battery. The power source may comprise a power management unit. The power management unit may be configured to convert the first voltage into a second voltage. The power source may comprise a charging coil. The charging coil may be provided by the magnetic antenna.

In an embodiment, the hearing device may comprise a memory, including volatile and nonvolatile forms of memory.

The hearing device may be a headset, a hearing aid, a hearable etc. The hearing device may be an in-the-ear (ITE) hearing device, a receiver-in-ear (RIE) hearing device, a receiver-in-canal (RIC) hearing device, a microphone-and-receiver-in-ear (MaRIE) hearing device, a behind-the-ear (BTE) hearing device comprising an ITE unit, or a one-size-fits-all hearing device etc.

The hearing device is configured to be worn by a user. The hearing device may be arranged at the user's ear, on the user's ear, in the user's ear, in the user's ear canal, behind the user's ear etc. The user may wear two hearing devices, one hearing device at each ear. The two hearing devices may be connected, such as wirelessly connected.

The hearing device may be configured for audio communication, e.g. enabling the user to listen to media, such as music or radio, and/or enabling the user to perform phone calls. The hearing device may be configured for performing hearing compensation for the user. The hearing device may be configured for performing noise cancellation etc.

The hearing device may comprise a RIE unit. The RIE unit typically comprises the earpiece such as a housing, a plug connector, and an electrical wire/tube connecting the plug connector and earpiece. The earpiece may comprise an in-the-ear housing, a receiver, such as a receiver configured for being provided in an ear of a user, and an open or closed dome. The dome may support correct placement of the earpiece in the ear of the user. The RIE unit may comprise an input transducer e.g. a microphone or a receiver, an output transducer e.g. an speaker, one or more sensors, and/or other electronics. Some electronic components may be placed in the earpiece, while other electronic components may be placed in the plug connector. The receiver may be with a different strength, i.e. low power, medium power, or high power. The electrical wire/tube provides an electrical connection between electronic components provided in the earpiece of the RIE unit and electronic components provided in the BTE unit. The electrical wire/tube as well as the RIE unit itself may have different lengths.

The hearing device may comprise an output transducer e.g. a speaker or receiver. The output transducer may be a part of a printed circuit board (PCB) of the hearing device. The output transducer may be arranged on a printed circuit board (PCB) of the hearing device. The output transducer may not be a part of the PCB of the hearing device. The output transducer may be configured to be arranged on the PCB of the hearing device. For instance, the output transducer may be configured to be arranged on an allocated position/area on the PCB of the hearing device. The output transducer may be arranged through a hole in the PCB.

The hearing device may comprise a first input transducer, e.g. a microphone, to generate one or more microphone output signals based on a received audio signal. The audio signal may be an analogue signal. The microphone output signal may be a digital signal. Thus, the first input transducer, e.g. microphone, or an analogue-to-digital converter, may convert the analogue audio signal into a digital microphone output signal. All the signals may be sound signals or signals comprising information about sound.

The hearing device may comprise a signal processor. The one or more microphone output signals may be provided to the signal processor for processing the one or more microphone output signals. The signals may be processed such as to compensate for a user's hearing loss or hearing impairment. The signal processor may provide a modified signal. All these components may be comprised in a housing of an ITE unit or a BTE unit. The hearing device may comprise a receiver or output transducer or speaker or loudspeaker. The receiver may be connected to an output of the signal processor. The receiver may output the modified signal into the user's ear. The receiver, or a digital-to-analogue converter, may convert the modified signal, which is a digital signal, from the processor to an analogue signal. The receiver may be comprised in an ITE unit or in an earpiece, e.g. RIE unit or MaRIE unit. The hearing device may comprise more than one microphone, and the ITE unit or BTE unit may comprise at least one microphone and the RIE unit may also comprise at least one microphone.

The hearing device signal processor may comprise elements such as an amplifier, a compressor and/or a noise reduction system etc. The signal processor may be implemented in a signal-processing chip or on the PCB of the hearing device. The hearing device may further have a filter function, such as compensation filter for optimizing the output signal.

The hearing device may comprise one or more antennas for radio frequency communication. The one or more antenna may be configured for operation in ISM frequency band. One of the one or more antennas may be an electric antenna. One or the one or more antennas may be a magnetic induction coil antenna. Magnetic induction, or near-field magnetic induction (NFMI), typically provides communication, including transmission of voice, audio and data, in a range of frequencies between 2 MHz and 15 MHz. At these frequencies the electromagnetic radiation propagates through and around the human head and body without significant losses in the tissue.

The magnetic induction coil may be configured to operate at a frequency below 100 MHz, such as at below 30 MHz, such as below 15 MHz, during use. The magnetic induction coil may be configured to operate at a frequency range between 1 MHz and 100 MHz, such as between 1 MHz and 15 MHz, such as between 1MHz and 30 MHz, such as between 5 MHz and 30 MHz, such as between 5 MHz and 15 MHz, such as between 10 MHz and 11 MHz, such as between 10.2 MHz and 11 MHz. The frequency may further include a range from 2 MHz to 30 MHz, such as from 2 MHz to 10 MHz, such as from 2 MHz to 10 MHz, such as from 5 MHz to 10 MHz, such as from 5 MHz to 7 MHz.

The electric antenna may be configured for operation at a frequency of at least 400 MHz, such as of at least 800 MHz, such as of at least 1 GHz, such as at a frequency between 1.5 GHz and 6 GHz, such as at a frequency between 1.5 GHz and 3 GHz such as at a frequency of 2.4 GHz. The antenna may be optimized for operation at a frequency of between 400 MHz and 6 GHz, such as between 400 MHz and 1 GHz, between 800 MHz and 1 GHz, between 800 MHz and 6 GHz, between 800 MHz and 3 GHz, etc. Thus, the electric antenna may be configured for operation in ISM frequency band. The electric antenna may be any antenna capable of operating at these frequencies, and the electric antenna may be a resonant antenna, such as monopole antenna, such as a dipole antenna, etc. The resonant antenna may have a length of A/4±10% or any multiple thereof, λbeing the wavelength corresponding to the emitted electromagnetic field.

The hearing device may comprise one or more wireless communications unit(s) or radios. The one or more wireless communications unit(s) are configured for wireless data communication, and in this respect interconnected with the one or more antennas for emission and reception of an electromagnetic field. Each of the one or more wireless communication unit may comprise a transmitter, a receiver, a transmitter-receiver pair, such as a transceiver, and/or a radio unit. The one or more wireless communication units may be configured for communication using any protocol as known for a person skilled in the art, including Bluetooth, WLAN standards, manufacture specific protocols, such as tailored proximity antenna protocols, such as proprietary protocols, such as low-power wireless communication protocols, RF communication protocols, magnetic induction protocols, etc. The one or more wireless communication units may be configured for communication using same communication protocols, or same type of communication protocols, or the one or more wireless communication units may be configured for communication using different communication protocols.

The wireless communication unit may connect to the hearing device signal processor and the antenna, for communicating with one or more external devices, such as one or more external electronic devices, including at least one smart phone, at least one tablet, at least one hearing accessory device, including at least one spouse microphone, remote control, audio testing device, etc., or, in some embodiments, with another hearing device, such as another hearing device located at another ear, typically in a binaural hearing device system.

The hearing device may be a binaural hearing device. The hearing device may be a first hearing device and/or a second hearing device of a binaural hearing device.

The hearing device may be a device configured for communication with one or more other device, such as configured for communication with another hearing device or with an accessory device or with a peripheral device..

The present invention relates to different aspects including the hearing device system, the hearing device, and the method described above and in the following, and corresponding systems, devices and methods, each yielding one or more of the benefits and advantages described in connection with the first mentioned aspect, and each having one or more embodiments corresponding to the embodiments described in connection with the first mentioned aspect and/or disclosed in the appended claims.

### BRIEF DESCRIPTION OF THE DRAWINGS

The above and other features and advantages will become readily apparent to those skilled in the art by the following detailed description of exemplary embodiments thereof with reference to the attached drawings, in which:
Fig. 1a schematically illustrates an exemplary hearing device system 2.
Fig. 1b schematically illustrates an exemplary hearing device 20 for compensating for a hearing loss of a user 4.
Fig. 2 schematically illustrates an exemplary method 100 for testing a user's hearing capabilities for providing a hearing threshold curve representing the user's hearing capabilities for use in a hearing device for the user.
Fig. 3 schematically illustrates an exemplary "Bayesian pure-tone audiometry" method for determining a user's hearing threshold (HT).
Fig. 4 schematically illustrates an exemplary simulation of estimation of a hearing threshold curve.
Fig. 5 schematically illustrates an exemplary flowchart for a method according to the present invention.
Fig. 6 shows the accuracy of the BPTA method as a function of iterations in audiogram space and gain space without taking the improved user experience into account (dark curve) and taken the user experience into account (bright curve).
Fig. 7 shows the consecutive number of non-heard tones, percentage of less than 4 consecutive non-heard tones, as a function of number of iterations, simply a measure of waiting time.

### DETAILED DESCRIPTION

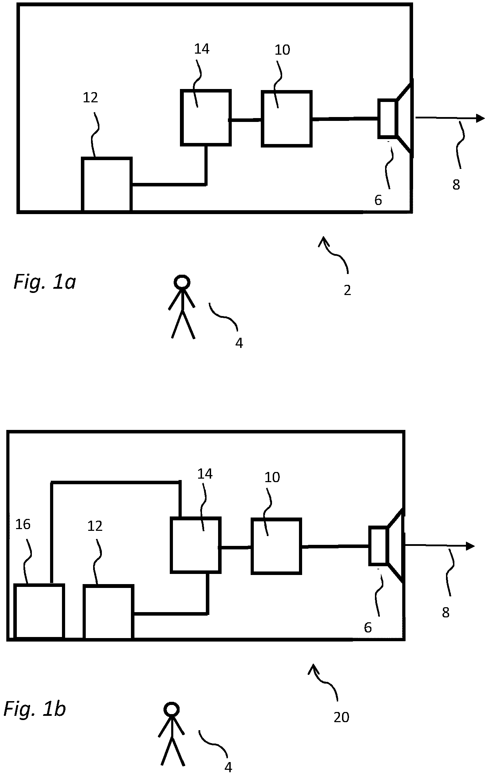

Various embodiments are described hereinafter with reference to the figures. Like reference numerals refer to like elements throughout. Like elements will, thus, not be described in detail with respect to the description of each figure. It should also be noted that the figures are only intended to facilitate the description of the embodiments. They are not intended as an exhaustive description of the claimed invention or as a limitation on the scope of the claimed invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described. Fig. 1a schematically illustrates an exemplary hearing device system 2. The hearing device system 2 is for performing a test of a hearing device user's 4 hearing capabilities for providing a hearing threshold curve representing the user's 4 hearing capabilities to be used for compensating for a hearing loss of the user 4. The hearing device system 2 comprises an acoustic output transducer 6 for outputting audio signals 8 in the ear of the user 4. The hearing device system 2 comprises a tone generator 10 for outputting a plurality of tones in the acoustic output transducer 6. A first set of the plurality of tones will be audible to the user 4 and a second set of the plurality of tones will be inaudible to the user 4. The hearing device system 2 comprises a user interface 12 configured for receiving an indication from the user 4 when a tone is audible. The hearing device system 2 comprises a processing unit 14 configured for: adaptively selecting the plurality of tones, and adaptively selecting an order of the plurality of tones. The adaptive selection of a new tone of the plurality of tones is based on: whether a predefined number of the preceding tones were audible or inaudible to the user 4, and an estimated probability of whether the new tone is audible or inaudible to the user 4.

Fig. 1b schematically illustrates an exemplary hearing device 20 for compensating for a hearing loss of a user 4. The hearing device 20 is configured to be worn in an ear of the user 4. The hearing device 20 is further configured for performing a test of the user's 4 hearing capabilities for providing a hearing threshold curve representing the user's 4 hearing capabilities to be used for the hearing compensation. The hearing device 20 comprises an acoustic output transducer 6 for outputting audio signals 8 in the ear of the user 4. The hearing device 20 comprises a tone generator 10 for outputting a plurality of tones in the acoustic output transducer 6. A first set of the plurality of tones will be audible to the user 4 and a second set of the plurality of tones will be inaudible to the user 4. The hearing device 20 comprises a user interface 12 and/or or a communication unit 16 for receiving an indication from the user 4 when a tone is audible. The hearing device 20 comprises a processing unit 14 configured for: adaptively selecting the plurality of tones, and adaptively selecting an order of the plurality of tones. The adaptive selection of a new tone of the plurality of tones is based on: whether a predefined number of the preceding tones were audible or inaudible to the user 4, and an estimated probability of whether the new tone is audible or inaudible to the user 4.

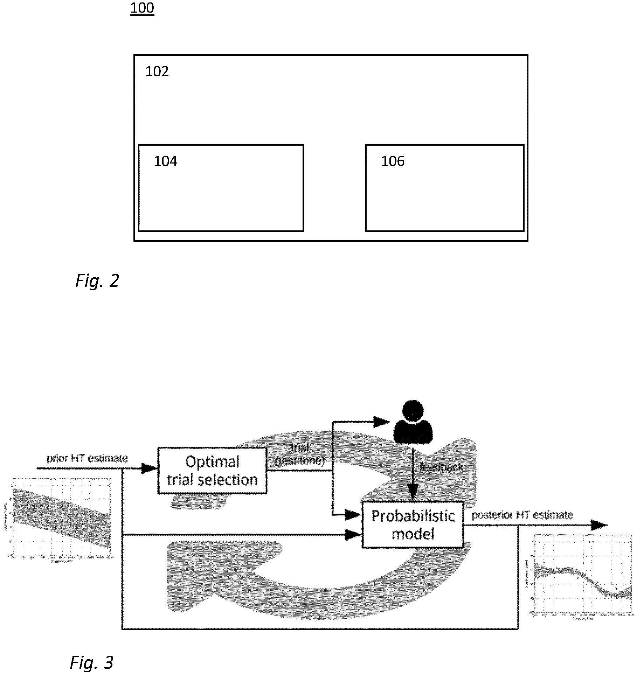

Fig. 2 schematically illustrates an exemplary method 100 for testing a user's hearing capabilities for providing a hearing threshold curve representing the user's hearing capabilities for use in a hearing device for the user. The method being performed in the hearing device system. The hearing device system comprises an acoustic output transducer for outputting audio signals in the ear of the user. The hearing device system comprises a tone generator for outputting a plurality of tones in the acoustic output transducer. A first set of the plurality of tones will be audible to the user and a second set of the plurality of tones will be inaudible to the user. The hearing device system comprises a user interface configured for receiving an indication from the user when a tone is audible. The hearing device system comprises a processing unit configured for: adaptively selecting the plurality of tones, and adaptively selecting an order of the plurality of tones. The method comprises performing 102 an adaptive selection of a new tone of the plurality of tones based on: whether 104 a predefined number of the preceding tones were audible or inaudible to the user, and an estimated probability 106 of whether the new tone is audible or inaudible to the user.

Fig. 3 schematically illustrates an exemplary "Bayesian pure-tone audiometry" method for determining a user's hearing threshold (HT). The present invention takes a probabilistic approach to the pure-tone hearing threshold estimation problem. One can view the threshold estimation problem as a probabilistic inference problem. To that end, we put a Gaussian process prior on the hearing threshold curve. Moreover, according to the present invention, the user's responses to stimuli may be modelled by a probabilistic response model. This model describes how the user response depends on their hearing threshold and the presented test tone. Given the response model, approximate Bayesian inference techniques may be applied to approximate the posterior distribution of the hearing threshold. This may result in a "Bayesian pure-tone audiometry" method illustrated by fig. 3. The Bayesian pure-tone audiometry method may incrementally decrease the uncertainty about the users hearing threshold curve by repeating the following steps:
1. Use the current (uncertain) estimate of the HT curve to determine the 'optimal' (most informative) next test tone. This tone may or may not be restricted to a set of standard frequencies.
2. Play the test tone and collect the user's 4 binary response: audible or non-audible.
3. Update the probabilistic estimate of the HT curve (i.e. approximate the posterior GP) based on the data collected so far.

These steps may be repeated until the uncertainty about the hearing threshold is sufficiently small. The fully probabilistic treatment of the estimation problem has some favourable properties:
An objective, information-theoretic criterion can be used to pick test tones such that the uncertainty is decreased as fast as possible. This minimizes the required number of trials to reach the desired accuracy.
Prior knowledge about the user's hearing threshold - for example based on age or medical history - can be leveraged to further decrease the number of required trials.
The complete threshold curve is inferred directly, instead of only inferring the thresholds at a set of standard frequencies.

The stopping criterium can be defined in an objective manner.

The method can be combined with other threshold estimation methods that take a probabilistic HT estimate as input.

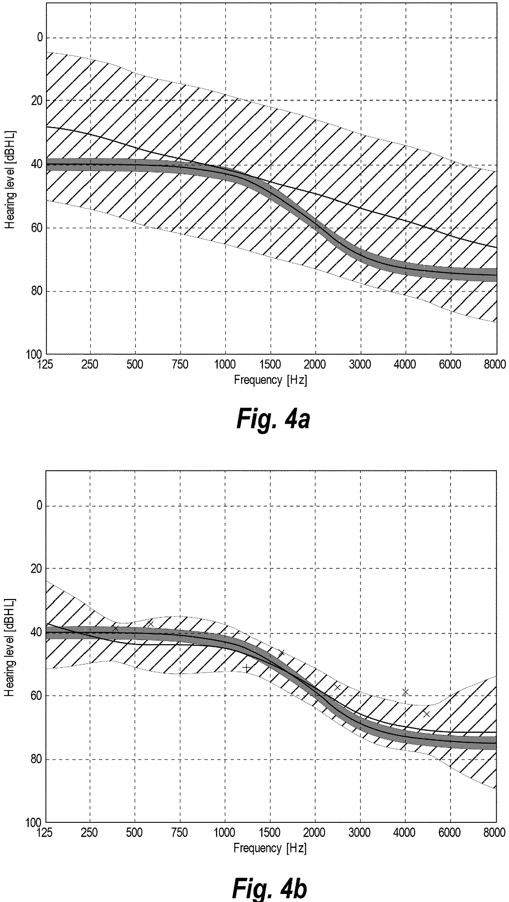

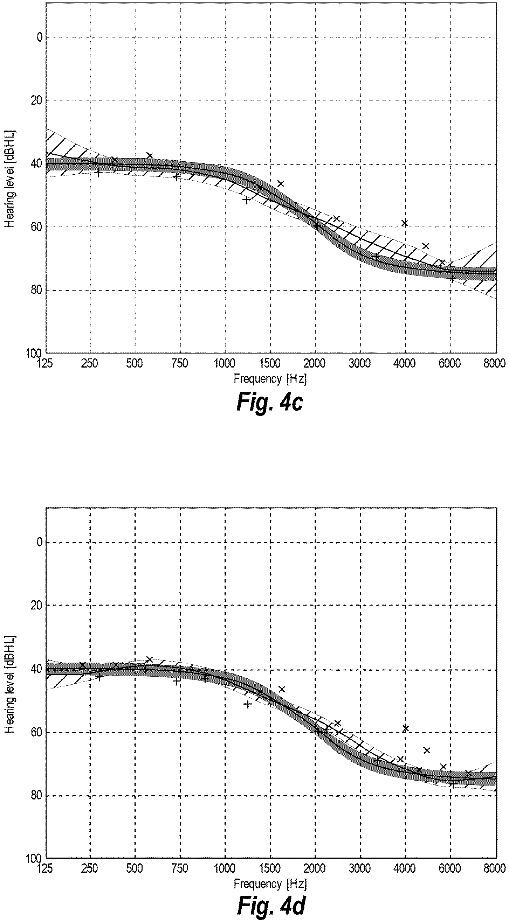

Fig. 4 schematically illustrates an exemplary simulation of estimation of a hearing threshold curve. Simulations have been performed in which it is shown to estimate an artifical hearing threshold curve. Fig. 4 depicts an example of the resulting 'probabilistic' audiograms after 7 (4b), 14 (4c), and 21 (4d) trials. Fig. 4a shows an audiogram based on a prior hearing threshold (HT) estimate. The narrow grey curve depicts the 'true' hearing threshold curve and the grey area covers 2 standard deviations of the user's response noise. The dark plusses and grey crosses represent test tones that were labelled as either audible (dark) or non-audible (grey). The grey line is the HT estimate based on the data so far, and the grey area depicts 2 standard deviations of the estimate uncertainty. The rate of convergence may obviously depend on the amount of noise in the response model. However, the method is robust in the sense that if the response model is sufficiently accurate, it will converge to the true hearing threshold curve in a minimum number of steps.

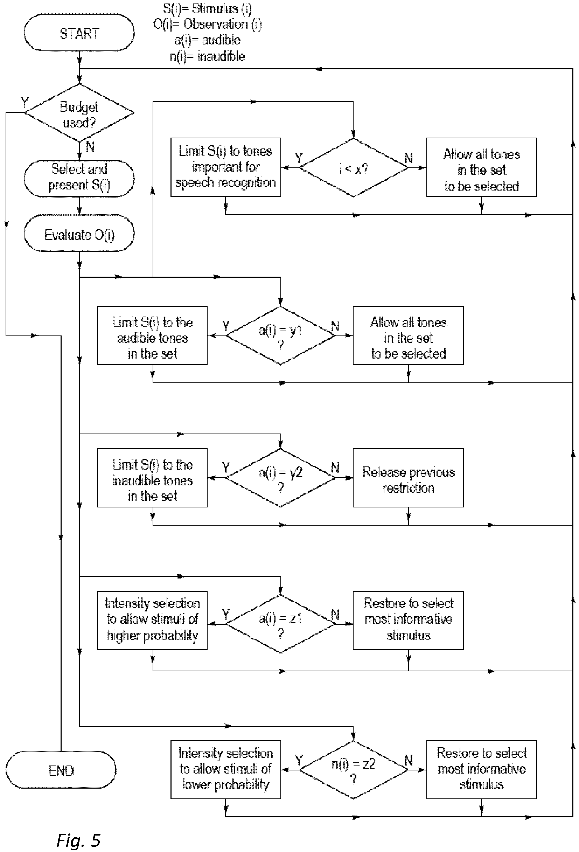

Fig. 5 schematically illustrates an exemplary flowchart for a method according to the present invention.

The method in fig. 5 may be termed a "BPTA method" and it is intelligent in the way that it may always choose the frequency and sound intensity that will provide the most information regarding converging to the true hearing threshold curve of the person under test based on all previous trials.

The 'standard audiometric frequencies' may be a set of standardized frequencies that are common in audiometric use, hence: 125, 250, 500, 750, 1000, 1250, 1500, 2000, 3000, 4000, 6000 and 8000 Hz. A subset of these frequencies may be selected prior to performing the BPTA method.

The first task of the BPTA method may be to adjust the probability that a tone will be audible given the estimate of the hearing threshold, which affects the intensity of the suggested tones. By default, this may be set to '0', which indicates to use intensities that match the estimated hearing threshold, the estimate being derived, e.g., from an audiogram taken prior to performing the BPTA method.

There may be two parts of the BPTA process, and these are described in the following. The first part may address the 'housekeeping tasks", e.g. ensuring that the user types in information related to age and gender prior to the audiometric process. This may provide the starting point of the audiometric process. A corresponding audiogram may be chosen based on age and gender if a personal audiogram is not available. This information may be extracted from a Gaussian modelling process on a large database, such as comprising about 80000 audiograms. The second part may address probabilities of the hearing thresholds for each of the subset of standardized frequencies. By default, the BPTA method may always propose a signal level near the expected hearing threshold of that person under test for a certain frequency. However, with the present improved user experience (UX) method, different probabilities can be selected. If the probability is set low, the algorithm may skew the suggested signal level towards the inaudible part of the hearing threshold. If the probability is set high, it may skew the suggested signal level towards the audible part of the hearing threshold. Note that the BPTA method may gain more new information the closer to the hearing threshold tones are tested. Therefore, there may always be the trade-off to consider of gaining new information with new tones but at the same time maintaining a good user experience, hence a good balance between heard and non-heard tones. Such trade-off was empirically evaluated by the applicant and led to the examples of numbers below.

The recommended settings for a specific hearing device may be:
- x =10,
- y1 = z1 = 5 and a corresponding audibility probability of 15%,
- y2 = z2 = 2 and a corresponding audibility probability of 85%.

The different ways of manipulating these mechanisms may be referred to as 'mode-switching'. Three strategies for this have been implemented in applications used for pilot testing by the applicant and can be applied at the same time:
The modes may be defined in the five variables x, y1, z1, y2 and z2. In general, the modes may refer to:
   - Default mode, i.e., BPTA may be working as described in the original reference of the method described in Cox, M. and de Vries, B. 2016.
   - Improved UX mode, i.e., one or more of the three strategies may be active with the goal to improve user experience without compromising on accuracy.
A first mode-switching strategy may cover variable x:
   For the first x tones the focus may be on those frequencies important for speech, e.g 1000-4000Hz. This restriction may be released at the x+1st tone.
   Hence, the algorithms may only select the frequencies 1000, 1500, 2000, 3000 or 4000 Hz. As stated, the purpose may be to start with frequencies that are the most important for speech perception.
A second mode-switching strategy may cover the variables y1 and z1 and may address the situation where there are too many consecutive audible tones:
   In order to "try to force an inaudible tone", the audibility probability of the proposed tones may be adjusted to 15% after y1 consecutive, audible tones, and the subset of frequencies which the method is allowed to propose may be reduced to only those frequencies for which an inaudible response has not yet been observed after z1 consecutive audible tones.
A third mode-switching strategy may cover the variables y2 and z2 and may address the situation when there are too many consecutive inaudible tones:
   In order to "try to force an audible tone", the audibility probability of the proposed tones may be adjusted to 85% after y2 consecutive, inaudible tones, and the subset of frequencies which the method is allowed to propose may be reduced to those frequencies for which an audible response has not yet been observed after z2 consecutive inaudible tones.

For the first x tones the focus may be on those frequencies important for speech, e.g. 1000-4000Hz. This restriction may be released at the x+1st tone.

The term 'focus' may imply that "the first x tones has to have a frequency in the range 1000-4000 Hz". Thus, the BPTA system may be required to propose tones with frequencies in the range 1000-4000 Hz in the first x steps. After x tones, this requirement may be lifted so tones with lower or higher frequencies are also allowed. This requirement ensures that the hearing threshold level may be first estimated at the frequencies that are important for speech.

After y1 number of audible tones, all frequencies may be removed from the set allowed for frequency selection for which no inaudible responses have been recorded. The frequency set may, however, be restored to all frequencies configured for the process after an inaudible response has been obtained.

The value of the parameter y1 may be independent of the method description. For a specific hearing device, the recommended value of y1=5 may be based on empirical optimization through a set of simulations. The applicant found that setting y1=5 yielded the best performance in the simulations. During the simulations, this value provided a good trade-off between triggering the method when it was beneficial while not triggering it too often.

Frequencies may be removed from the set if no inaudible responses have been recorded. The main reason being that since those frequencies can be heard by the user it is unnecessary to repeat them, thus saving time. In this context, "the set" may relate to the set of frequencies that BPTA to choose from to propose the next test tone. The method may keep track of a set of frequencies that BPTA can choose from when proposing the next tone to test. If a frequency is removed from the set, BPTA may not propose tones with that frequency.

By default, the frequency set, i.e. the set of frequencies that BPTA can choose from when proposing tones, may contain the standard audiometric frequencies. However, the various mechanisms in the described method may adaptively add or remove frequencies from this set. If the frequency set is restored, it may be set back to the default (= standard audiometric frequencies).

After y2 number of inaudible tones all frequencies may be removed from the set allowed for frequency selection for which no audible responses have been recorded. The main reason being that since those frequencies cannot be heard by the user it is unnecessary to repeat them, thus saving time. The frequency set may be restored to all frequencies configured for the process after an audible response has been obtained.

The parameter y2 may be chosen in a manner similar to y1, through empirical optimization by simulations. In general, the parameters can be chosen by hand, optimized through simulations, or optimized through real-world experiments.

After z1 number of audible tones, the 'audibility probability' may be reduced closer to 0%, but not at 0%, to try and force an inaudible response. Once an inaudible response has been obtained, the audibility probability may be reset to the default selected for the process.

Both values of parameters z1 and z2 may be obtained through empirical optimization. They do not need to be equal, but testing has revealed that setting them to the same value provides the best performance. This is expected since both parameters determine when a mechanism is triggered to "force" an audible or inaudible tone.

After z2 number of inaudible tones, the 'audibility probability' may be increased closer to 100% to try and force an audible response. Once an audible response has been obtained, the audibility probability may be reset to the default probability selected for the process. This is also empirically determined. The audibility probabilities related to "inaudible" and "audible" tones are informed choices to increase the likelihood of the tone being evaluated at the desired audibility while still providing information to the system. Setting these to, say, 1% and 100% respectively, would make it very unlikely for the system to learn. The default setting of "the estimated threshold", may be the "most informative". The further evaluated tones deviate from this value, the less "informative" they may be.

The method is a tradeoff between accuracy and user experience given a fixed budget of stimuli, i.e., how many stimuli is a user willing to evaluate during a session. Typically, the same accuracy can also be acquired by extending the budget. However, that may arguably decrease the user experience as the procedure requires more investment from the user regarding time, attention, and patience. These methods are specifically designed for automated procedures in situations where a limited time investment is targeted to be required from an end-user, but where the completion of the procedure is a necessity for accurate functioning of systems further down the pipeline, e.g. the determination of compressor settings in a hearing instrument. In user trials, the applicant has observed people to get bored/disinterested when they don't hear enough variation in the tones, e.g. they always hear a tone, or they start doubting whether the system even works because they never hear a tone.

With the present method it is an aim to have a good balance between heard and non-heard tones such that the user has a satisfying experience, e.g. not too long pauses, and at the same time not compromising accuracy in the method itself. The improvement can be explained by a plot. Consider figures 6 and 7.

Figure 6 shows the accuracy of the BPTA method as a function of iterations in audiogram space (left column) and gain space (right column), without taking the improved user experience into account (red curve) and taken the user experience into account (bright curve). The accuracy is unaltered in both ways.

Figure 6 shows accuracy as a function of iterations in audiogram space (left column) and gain space (right column) for simulations (top row) and a listening study (called TRL3) being an in silence listening study (bottom row).

Top left: Accuracy simulated in audiogram space as a function of iterations. Current settings of BPTA are reflected by the bright curve. The settings include the mode-switch and mid-frequency trials to improve the user-experience (UX). The dark curve represents performance when the UX improvements are disabled. The UX improvements do not impair the overall accuracy.

Top Right: Accuracy simulated in gain space as a function of iterations. Above 20 iterations the UX improvements do not impair the accuracy performance, but slight drawbacks are observed below 20 iterations.

Bottom left: Analysis of audiogram space for 11 participants in the TRL3 in silence study. Here 30 iterations are needed to have 90% of the audiograms within the success-criterium.

Bottom right: Analysis of gain space for 11 participants in the TRL3 in silence study. Here 20 iterations are needed to have 100% of the audiograms within the success-criterium of +-5 dB difference in gain.

Fig. 7 shows that the user experience is significantly improved. Figure 7 shows the consecutive number of non-heard tones, percentage of less than 4 consecutive non-heard tones, as a function of number of iterations, simply a measure of waiting time. A higher value is desirable here, ensuring that there are not too long pauses due to non-heard tones. Here the bright curve, being the user experience improvement, overrules the dark curve, being neglecting the user experience, suggesting that the present method provides the desired user experience.

Figure 7 shows consecutive number of non-heard tones (percentage of less than 4 consecutive non-heard tones) as a function of number of iterations. A higher value is desirable here ensuring that there are not too long pauses of non-heard tones.

Left: Simulation-based analysis. The bright line shows the effectiveness of having less non-heard tones compared to when this feature is turned off (dark line).

Right: TRL3 in silence study results match well the predicted performance from simulations.

Although particular features have been shown and described, it will be understood that they are not intended to limit the claimed invention, and it will be made obvious to those skilled in the art that various changes and modifications may be made without departing from the scope of the claimed invention. The specification and drawings are, accordingly to be regarded in an illustrative rather than restrictive sense. The claimed invention is intended to cover all alternatives, modifications and equivalents.

### ITEMS:

1. A hearing device system for performing a test of a hearing device user's hearing capabilities for providing a hearing threshold curve representing the user's hearing capabilities to be used for compensating for a hearing loss of the user, where the hearing device system comprises:
   - an acoustic output transducer for outputting audio signals in the ear of the user;
   - a tone generator for outputting a plurality of tones in the acoustic output transducer; where a first set of the plurality of tones will be audible to the user and a second set of the plurality of tones will be inaudible to the user;
   - a user interface configured for receiving an indication from the user when a tone is audible;
   - a processing unit configured for:
      - adaptively selecting the plurality of tones; and
      - adaptively selecting an order of the plurality of tones; wherein the adaptive selection of a new tone of the plurality of tones is based on:
      - whether a predefined number of the preceding tones were audible or inaudible to the user; and
      - an estimated probability of whether the new tone is audible or inaudible to the user.
2. The hearing device system according to any of the preceding items, wherein the adaptive selection of a new tone is based on between one and five preceding tones, preferably two preceding tones, being inaudible to the user before a probable audible tone is selected.
3. The hearing device system according to any of the preceding items, wherein the adaptive selection of a new tone is based on between two and eight preceding tones, preferably five preceding tones, being audible to the user before a probable inaudible tone is selected.
4. The hearing device system according to any of the preceding items, wherein the estimated probability of whether the new tone is audible to the user is between about 75% and about 95%, preferably about 85%, probability.
5. The hearing device system according to any of the preceding items, wherein the estimated probability of whether the new tone is inaudible to the user is between about 5% and about 25%, preferably about 15% probability.
6. The hearing device system according to any of the preceding items, wherein the plurality of tones are selected from a set of audiometric frequencies in a range from about 200 Hz to about 10 kHz, preferably including the frequencies 250 Hz, 500 Hz, 750 Hz, 1000 Hz, 1500 Hz, 2000 Hz, 3000 Hz, 4000 Hz, 6000 Hz, and 8000 Hz.
7. The hearing device system according to any of the preceding items, wherein the plurality of tones in a mid-frequency trial of the test are 10 tones selected from a subset of audiometric frequencies important for speech in the range from about 1kHz to about 4 kHz, preferably including the frequencies 1000 Hz, 1500 Hz, 2000 Hz, 3000 Hz, 4000 Hz.
8. The hearing device system according to any of the preceding items, wherein the plurality of tones in the complete test is dependent of a predetermined confidence interval of the test, preferably at least 20 tones.
9. The hearing device system according to any of the preceding items, wherein the hearing threshold curve representing the user's hearing capabilities is estimated by Bayesian pure tone audiometry (BPTA).
10. The hearing device system according to any of the preceding items, wherein the Bayesian pure tone audiometry (BPTA) comprises a probabilistic approach to estimate the hearing threshold of the user based on a probabilistic model of the hearing threshold curve, consisting of a weighted combination of Gaussian process components that represent typical hearing threshold curves, the components being trained on a database of hearing threshold curves, where information including the user's age and gender is used to compute the underlying weights of the components, and where the BPTA estimates the user's hearing threshold curves as a smooth continuous function of frequency versus hearing threshold.
11. The hearing device system according to any of the preceding items, wherein the hearing device system is configured for performing the test of the user's hearing capabilities for providing the hearing threshold curve representing the user's hearing capabilities by initiation from the user and without assistance from a hearing care professional.
12. The hearing device system according to any of the preceding items, wherein the processing unit is configured for generating the hearing threshold curve representing the user's hearing capabilities to be used for the hearing compensation when the plurality of tones has been outputted according to the adaptive selection.
13. The hearing device system according to any of the preceding items, wherein the hearing device system comprises a hearing device configured to be worn in an ear of the user for compensating for the hearing loss of the user, and wherein the hearing device comprises the acoustic output transducer for outputting audio signals in the ear of the user.
14. The hearing device system according to any of the preceding items, wherein the processing unit is a first processing unit of the hearing device or a second processing unit of an electronic device.
15. The hearing device system according to any of the preceding items, wherein the user interface is provided at the hearing device, at an auxiliary device, and/or at an electronic device comprising a communication unit for communicating with a hearing device system communication unit.
16. A hearing device for compensating for a hearing loss of a user, the hearing device is configured to be worn in an ear of the user, where the hearing device is further configured for performing a test of the user's hearing capabilities for providing a hearing threshold curve representing the user's hearing capabilities to be used for the hearing compensation, where the hearing device comprises:
   - an acoustic output transducer for outputting audio signals in the ear of the user;
   - a tone generator for outputting a plurality of tones in the acoustic output transducer; where a first set of the plurality of tones will be audible to the user and a second set of the plurality of tones will be inaudible to the user;
   - a user interface or a communication unit for receiving an indication from the user when a tone is audible;
   - a processing unit configured for:
      - adaptively selecting the plurality of tones; and
      - adaptively selecting an order of the plurality of tones; wherein the adaptive selection of a new tone of the plurality of tones is based on:
      - whether a predefined number of the preceding tones were audible or inaudible to the user; and
      - an estimated probability of whether the new tone is audible or inaudible to the user.
17. A method for testing a user's hearing capabilities for providing a hearing threshold curve representing the user's hearing capabilities for use in a hearing device for the user, the method being performed in the hearing device system comprising:
   - an acoustic output transducer for outputting audio signals in the ear of the user,
   - a tone generator for outputting a plurality of tones in the acoustic output transducer; where a first set of the plurality of tones will be audible to the user and a second set of the plurality of tones will be inaudible to the user;
   - a user interface configured for receiving an indication from the user when a tone is audible;
   - a processing unit configured for:
      - adaptively selecting the plurality of tones; and
      - adaptively selecting an order of the plurality of tones; wherein the method comprises:
         - performing an adaptive selection of a new tone of the plurality of tones based on:
            - whether a predefined number of the preceding tones were audible or inaudible to the user; and
            - an estimated probability of whether the new tone is audible or inaudible to the user.

### LIST OF REFERENCES

2 hearing device system
4 user
6 acoustic output transducer
8 audio signals
10 tone generator
12 user interface
14 processing unit
20 hearing device
100 method for testing a user's hearing capabilities for providing a hearing threshold curve representing the user's hearing capabilities for use in a hearing device for the user;
201 method step of performing an adaptive selection of a new tone of the plurality of tones based on: whether 104 a predefined number of the preceding tones were audible or inaudible to the user, and an estimated probability 106 of whether the new tone is audible or inaudible to the user.

## Claims

1. A hearing device system for performing a test of a hearing device user's hearing capabilities for providing a hearing threshold curve representing the user's hearing capabilities to be used for compensating for a hearing loss of the user, where the hearing device system comprises:
- an acoustic output transducer for outputting audio signals in the ear of the user;
- a tone generator for outputting a plurality of tones in the acoustic output transducer; where a first set of the plurality of tones will be audible to the user and a second set of the plurality of tones will be inaudible to the user;
- a user interface configured for receiving an indication from the user when a tone is audible;
- a processing unit configured for:
- adaptively selecting the plurality of tones; and
- adaptively selecting an order of the plurality of tones; wherein the adaptive selection of a new tone of the plurality of tones is based on:
- whether a predefined number of the preceding tones were audible or inaudible to the user; and
- an estimated probability of whether the new tone is audible or inaudible to the user.

2. The hearing device system according to any of the preceding claims, wherein the adaptive selection of a new tone is based on between one and five preceding tones, preferably two preceding tones, being inaudible to the user before a probable audible tone is selected.

3. The hearing device system according to any of the preceding claims, wherein the adaptive selection of a new tone is based on between two and eight preceding tones, preferably five preceding tones, being audible to the user before a probable inaudible tone is selected.

4. The hearing device system according to any of the preceding claims, wherein the estimated probability of whether the new tone is audible to the user is between about 75% and about 95%, preferably about 85%, probability.

5. The hearing device system according to any of the preceding claims, wherein the estimated probability of whether the new tone is inaudible to the user is between about 5% and about 25%, preferably about 15% probability.

6. The hearing device system according to any of the preceding claims, wherein the plurality of tones are selected from a set of audiometric frequencies in a range from about 200 Hz to about 10 kHz, preferably including the frequencies 250 Hz, 500 Hz, 750 Hz, 1000 Hz, 1500 Hz, 2000 Hz, 3000 Hz, 4000 Hz, 6000 Hz, and 8000 Hz.

7. The hearing device system according to any of the preceding claims, wherein the plurality of tones in a mid-frequency trial of the test are 10 tones selected from a subset of audiometric frequencies important for speech in the range from about 1kHz to about 4 kHz, preferably including the frequencies 1000 Hz, 1500 Hz, 2000 Hz, 3000 Hz, 4000 Hz.

8. The hearing device system according to any of the preceding claims, wherein the plurality of tones in the complete test is dependent of a predetermined confidence interval of the test, preferably at least 20 tones.

9. The hearing device system according to any of the preceding claims, wherein the hearing threshold curve representing the user's hearing capabilities is estimated by Bayesian pure tone audiometry (BPTA).

10. The hearing device system according to any of the preceding claims, wherein the Bayesian pure tone audiometry (BPTA) comprises a probabilistic approach to estimate the hearing threshold of the user based on a probabilistic model of the hearing threshold curve, consisting of a weighted combination of Gaussian process components that represent typical hearing threshold curves, the components being trained on a database of hearing threshold curves, where information including the user's age and gender is used to compute the underlying weights of the components, and where the BPTA estimates the user's hearing threshold curves as a smooth continuous function of frequency versus hearing threshold.

11. A hearing device for compensating for a hearing loss of a user, the hearing device is configured to be worn in an ear of the user, where the hearing device is further configured for performing a test of the user's hearing capabilities for providing a hearing threshold curve representing the user's hearing capabilities to be used for the hearing compensation, where the hearing device comprises:
- an acoustic output transducer for outputting audio signals in the ear of the user;
- a tone generator for outputting a plurality of tones in the acoustic output transducer; where a first set of the plurality of tones will be audible to the user and a second set of the plurality of tones will be inaudible to the user;
- a user interface or a communication unit for receiving an indication from the user when a tone is audible;
- a processing unit configured for:
- adaptively selecting the plurality of tones; and
- adaptively selecting an order of the plurality of tones; wherein the adaptive selection of a new tone of the plurality of tones is based on:
- whether a predefined number of the preceding tones were audible or inaudible to the user; and
- an estimated probability of whether the new tone is audible or inaudible to the user.

12. A method for testing a user's hearing capabilities for providing a hearing threshold curve representing the user's hearing capabilities for use in a hearing device for the user, the method being performed in the hearing device system comprising:
- an acoustic output transducer for outputting audio signals in the ear of the user,
- a tone generator for outputting a plurality of tones in the acoustic output transducer; where a first set of the plurality of tones will be audible to the user and a second set of the plurality of tones will be inaudible to the user;
- a user interface configured for receiving an indication from the user when a tone is audible;
- a processing unit configured for:
- adaptively selecting the plurality of tones; and
- adaptively selecting an order of the plurality of tones;
wherein the method comprises:
- performing an adaptive selection of a new tone of the plurality of tones based on:
- whether a predefined number of the preceding tones were audible or inaudible to the user; and
- an estimated probability of whether the new tone is audible or inaudible to the user.
